# EUROPEAN PATENT APPLICATION

(11) **EP 1 310 553 A1**
(43) Date of publication of application: **14.05.2003**
(21) Application number: 01956912.8
(22) Date of filing: 16.08.2001
(51) Int. Cl.: C12N 15/09, C12Q 1/68

(54) **METHOD OF ESTIMATING THE GENOTYPE OF FERTILITY RECOVERY LOCUS TO RICE BT TYPE MALE FERTILITY CYTOPLASM**

(30) Priority: 17.08.2000 JP 2000247204
(71) Applicant: Japan Tobacco Inc., Tokyo 105-8422 (JP); Syngenta Limited, Guildford Surrey GU2 7YH (GB)
(72) Inventor: KOMORI, Toshiyuki, c/o JAPAN TOBACCO INC.,, Iwata-gun, Shizuoka 438-0802 (JP); YAMAMOTO, Toshiom, Chiba 292-0801 (JP); NITTA, Naoto, c/o JAPAN TOBACCO INC.,, Minato-ku, Tokyo 105-8422 (JP); TAKEMORI, Naoki, c/o Orynova K.K., Iwata-gun, Shizuoka 438-0802 (JP)
(74) Representative: Behnisch, Werner, Dr.
(86) International application number: JP0107052
(87) International publication number: WO02014506

(57) **Abstract**

The invention relates to a method of detecting Rf-1 gene, or the gene that restores the BT type cytoplasmic male sterility which is employed in hybrid rice breeding. More specifically, the invention provides a method of detecting Rf-1 gene by making use of the fact that a plurality of PCR markers present near the locus of Rf-1 gene are linked to said locus. Specifically, the invention genotypes a plurality of PCR marker loci present in the neighborhood of Rf-1 gene based on the fact that the locus of Rf-1 gene is located between novel PCR markers S12564 Tsp509I and C1361 MwoI on chromosome 10 of rice, whereby estimation of the presence or absence of Rf-1 gene and selection of Rf-1 gene homozygous individuals can be performed in a convenient and accurate way.

## Description

### Field of the Invention

This invention relates to a method of detecting Rf-1 gene in rice individuals. Rf-1 gene restores the BT type cytoplasmic male sterility, and hence, is currently used in the breeding of japonica-japonica hybrid rice.

More particularly, the invention relates to a method which, relying on the fact that the locus of Rf-1 gene is located between DNA markers S12564 Tsp509I and C1361 MwoI on chromosome 10 of rice, detects PCR markers present in the neighborhood of Rf-1 gene, whereby investigation for the presence or absence of Rf-1 gene and selection of Rf-1 gene homozygous individuals can be performed in a convenient and accurate way.

### Prior Art

Rice is a self-fertilizing plant, so in order to perform crossing between varieties, self-fertilization must first be avoided by removing all stamens in a glumaceous flower just before flowering and, then fertilization is effected with pollens from the parent variety with which it is to be crossed. However, this manual crossing method is entirely unsuitable for producing a large quantity of hybrid seeds on a commercial scale.

Alternatively, hybrid rice is produced by the triple-crossing system which makes use of cytoplasmic male sterility. In the triple-crossing system, the following three lines are employed, i.e., a sterile line having male sterile cytoplasm, a restorer line having Rf-1 gene and a maintainer line having the same nuclear gene as that of the sterile line but not having any sterile cytoplasm. By using these three lines, (i) hybrid seeds can be obtained through fertilization of the sterile line with the pollen of the restorer line whereas (ii) the sterile line can be maintained through its fertilization with the pollen of the maintainer line.

When employing the BT type male sterile cytoplasm in the triple-crossing system, it is important to breed rice of the restorer line and to this end, it is necessary to ensure that the rice at every stage of breeding maintains Rf-1 gene and that the Rf-1 gene is homozygous at the final stage. It also becomes necessary in the triple-crossing system to check to ensure that the variety used as the restorer line possesses Rf-1 gene, or to check for the presence of Rf-1 gene in order to ensure that the resulting hybrid seeds have restored fertility.

In order to genotype the locus of Rf-1 gene in a plant, it has been necessary that F1 plants be first formed from hybrid seeds obtained by crossing the plant to be genotyped to a standard line and then self-fertilized, followed by investigating the incidence of individuals that can produce seeds at a frequency higher than a certain level (e.g. ≥ 70∼80%). The standard line refers to the maintainer line, the sterile line or a set of the two lines, and it is appropriately chosen depending upon whether the cytoplasm of the individual under test is of BT type or normal type or unknown. If the standard line is a sterile line, it is crossed to the individual under test as the female parent and if the standard line is a maintainer line, it is crossed as the male parent.

However, these techniques require a huge amount of labor and time to carry out. As a further problem, fertilization for seed production is sensitive to environmental factors and if an investigation is made in an unfavorable environment such as cold climate or insufficient daylight, sterility may be caused irrespective of the genotype constitution, with the result that genotyping of the locus of Rf-1 gene cannot be performed accurately.

With a view to solving these problems, it has recently been proposed that Rf-1 gene be checked for its presence by a technique in molecular biology. The heart of this technique lies in checking for the presence or absence of Rf-1 gene by detecting nucleotide sequences linked to Rf-1 gene (such sequences are hereunder referred to as DNA markers). Note that it is not possible to directly detect Rf-1 gene since the DNA sequence of Rf-1 gene has not been clarified.

It has been reported that the locus of Rf-1 gene in rice is present on chromosome 10 and located between DNA marker (RFLP marker) loci G291 and G127 which can be used in restriction fragment length polymorphism analysis (RFLP) (Fukuta et al., 1992, Jpn J. Breed. 42 (supl. 1) 164-165). This is a known method of genotyping the locus of Rf-1 gene by investigating the genotypes of DNA marker loci G291 and G127 which are linked to Rf-1 gene.

However, the conventional molecular biology techniques have several problems. First, they use RFLP markers which need to be detected by Southern blot analysis. In order to perform Southern blot analysis, DNA at the microgram level needs to be purified from the individual under test and, in addition, there is a need to carry out a sequence of steps comprising treatment with restriction enzymes, electrophoresis, blotting, hybridization with a probe and signal detection; this not only involves considerable labor but it also takes about one week to obtain the test results.

The second problem is that since the gene map distance between RFLP marker loci G291 and G127 is as long as about 30 cM (corresponding to about 9000 kbp in rice DNA), the probability for the occurrence of double recombination in the region would be a few percent and hence, it is not always guaranteed that the genotype of the locus of Rf-1 gene can be estimated correctly by the markers.

Thirdly, when the presence of Rf-1 gene is estimated by detecting RFLP marker loci G291 and G127, not only Rf-1 gene but also the gene region between those loci are introduced into the fertility restorer line selected as the result of breeding. As a consequence, the introduced DNA sequence will have a chromosomal region of 30 cM or longer from the Rf-1 gene donor parent, and this presents the risk of introducing a deleterious gene that may potentially be present within that region.

In order to solve these problems, there have been developed a dominant DNA marker (Japanese Patent Laid-Open No. 222588/1995) and a co-dominant DNA marker (Japanese Patent Laid-Open No. 313187/1997), both of which are linked to the locus of Rf-1 gene. These markers are linked to the locus of Rf-1 gene, their genetic distances from Rf-1 gene respectively being 1.6 ± 0.7 cM (corresponding to about 480 kbp in rice DNA) and 3.7 ± 1.1 cM (corresponding to about 1110 kbp in rice DNA), and their loci being on opposite sides of the locus of Rf-1 gene. Hence, the presence of Rf-1 gene can be estimated by detecting the presence of both the locus of the dominant PCR marker and that of the co-dominant PCR marker. The use of the co-dominant PCR marker also enables to estimate as to whether the locus of Rf-1 gene is homozygous or heterozygous.

However, the use of these PCR markers still involve several problems. The co-dominant marker has a genetic distance of 3.7 ± 1.1 cM from the locus of Rf-1 gene, and the problem of potentially high frequency of recombination with the locus of Rf-1 gene has not been fully dissolved. Speaking of the co-dominant marker itself, correct detection can be made as to whether it is homozygous or a heterozygous. However, if recombination occurs between the locus of the co-dominant marker and that of Rf-1 gene, the genotype of Rf-1 gene locus cannot be determined correctly, particularly as to whether it is homozygous or heterozygous. On the other hand, if the dominant marker is used to genotype the locus of Rf-1 gene, the marker will detect individuals indiscriminately irrespective of whether they are homozygous (Rf-1/Rf-1) or heterozygous (Rf-1/rf-1) with respect to Rf-1 gene. Therefore, even if the co-dominant marker is used in combination with the dominant marker in order to genotype the locus of Rf-1 gene, it is not possible to correctly distinguish individuals having Rf-1 gene homozygously from those having the gene heterozygously. Further, if no amplification product is obtained in PCR using the dominant marker, one cannot deny the possibility that this is due to some problems in the experimental procedure. As a further problem, since the genetic distance between the co-dominant marker and the dominant marker is as great as about 5.3 cM (ca. 1590 kbp), the size of the chromosomal region introduced from the Rf-1 gene donor parent cannot be limited to a sufficiently small value to prevent any concomitant introduction of a deleterious gene which may be contained in that region.

Japanese Patent Laid-Open No. 139465/2000 describes co-dominant PCR markers that were developed on the basis of the nucleotide sequences of RFLP markers located in the neighborhood of Rf-1 gene on chromosome 10 of rice. However, most of those PCR markers are spaced from the Rf-1 gene by a genetic distance greater than about 1 cM.

### Summary of the Invention

An object of the invention is to provide a method by which the locus of Rf-1 gene can be detected and genotyped (i.e., whether it is heterozygous or homozygous) in a convenient and accurate way by using a plurality of newly developed co-dominant PCR markers that are closely linked to the locus of Rf-1 gene and which are positioned on opposite sides of the Rf-1 locus.

### Brief Description of the Drawing

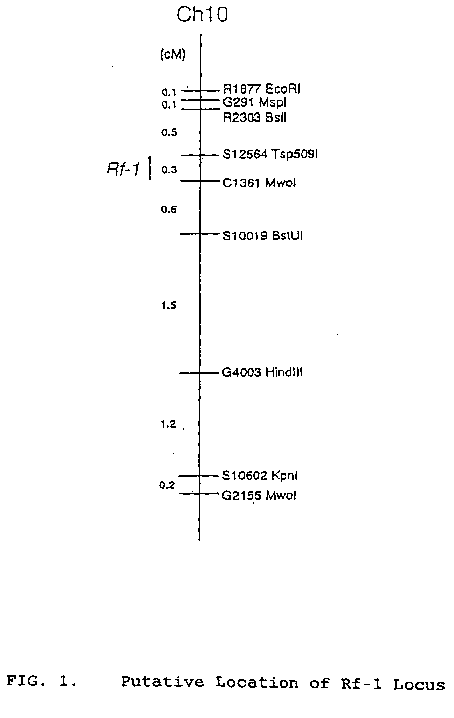

Fig. 1 is a gene map in which the locus of Rf-1 gene on chromosome 10 of rice is positioned on a linkage map in relation to various markers; the values of map distance were calculated from the segregation data from 1042 F1 individuals.

### Detailed Description of the Invention

The present invention relates to a method of detecting Rf-1 gene, or the gene that restores the BT type cytoplasmic male sterility which is employed in hybrid rice breeding. More particularly, the present inventors found that the locus of Rf-1 gene could be limited to within a very small region on chromosome 10 of rice. Based on this result, the present inventors developed PCR markers present in the neighborhood of the locus of Rf-1 gene. The invention provides a method of detecting Rf-1 gene by making use of the fact that those PCR markers are linked to the locus of Rf-1 gene. Specifically, the invention, relying on the fact that the locus of Rf-1 gene is located between PCR markers S12564 Tsp509I and C1361 MwoI on chromosome 10 of rice, detects the novel PCR markers present in the neighborhood of Rf-1 gene, whereby investigation for the presence or absence of Rf-1 gene and selection of Rf-1 gene homozygous individuals can be performed in a convenient and accurate way.

### Markers

Primer pairs designed to be specific to particular regions near the locus of Rf-1 gene are used in PCR and the amplification products are treated with particular restriction enzymes; upon electrophoresis, rice of *indica* lines in some cases provide an observable band of a different size from that of rice of *Japonica* lines. This band which is characteristic of *indica* lines is herein referred to as the Rf-1 linked band. Now that it has been made clear by the present invention that the locus of Rf-1 gene is located between PCR markers S12564 Tsp509I and C1361 MwoI on chromosome 10 of rice, the skilled artisan can appropriately develop and employ PCR markers that are present in the neighborhood of Rf-1 gene.

For instance, according to the invention, a rice individual under test is checked to see if its genome contains at least one of the PCR markers listed below, thereby determining whether the individual under test has Rf-1 gene linked to those PCR markers:
(1) marker 1: PCR marker R1877 EcoRI which, when rice genomic DNA is subjected to PCR with DNA primers having the sequences of SEQ ID NO:1 and SEQ ID NO:2, can detect polymorphisms between rice individuals of the *japonica* and *indica* lines depending on whether the amplification products have a recognition site for restriction enzyme EcoRI;
(2) marker 2: PCR marker G4003 HindIII (SEQ ID NO:19) which, when rice genomic DNA is subjected to PCR with DNA primers having the sequences of SEQ ID NO:3 and SEQ ID NO:4, can detect polymorphisms between rice individuals of the *japonica* and *indica* lines depending on whether the amplification products have a recognition site for restriction enzyme HindIII;
(3) marker 3: PCR marker C1361 MwoI (SEQ ID NO:20) which, when rice genomic DNA is subjected to PCR employing DNA primers having the sequences of SEQ ID NO:5 and SEQ ID NO:6, can detect polymorphisms between rice individuals of the *japonica* and *indica* lines depending on whether the amplification products have a recognition site for restriction enzyme MwoI;
(4) marker 4: PCR marker G2155 MwoI (SEQ ID NO:21) which, when rice genomic DNA is subjected to PCR with DNA primers having the sequences of SEQ ID NO:7 and SEQ ID NO:8, can detect polymorphisms between rice individuals of the *japonica* and *indica* lines depending on whether the amplification products have a recognition site for restriction enzyme MwoI;
(5) marker 5: PCR marker G291 MspI (SEQ ID NO:22) which, when rice genomic DNA is subjected to PCR with DNA primers having the sequences of SEQ ID NO:9 and SEQ ID NO:10, can detect polymorphisms between rice individuals of the *japonica* and *indica* lines depending on whether the amplification products have a recognition site for restriction enzyme MspI;
(6) marker 6: PCR marker R2303 Bs1I (SEQ ID NO:23) which, when rice genomic DNA is subjected to PCR with DNA primers having the sequences of SEQ ID NO:11 and SEQ ID NO:12, can detect polymorphisms between rice individuals of the *japonica* and *indica* lines depending on whether the amplification products have a recognition site for restriction enzyme Bs1I;
(7) marker 7: PCR marker S10019 BstUI (SEQ ID NO:24) which, when rice genomic DNA is subjected to PCR with DNA primers having the sequences of SEQ ID NO:13 and SEQ ID NO:14, can detect polymorphisms between rice individuals of the *japonica* and *indica* lines depending on whether the amplification products have a recognition site for restriction enzyme BstUI;
(8) marker 8: PCR marker S10602 KpnI (SEQ ID NO:25) which, when rice genomic DNA is subjected to PCR with DNA primers having the sequences of SEQ ID NO:15 and SEQ ID NO:16, can detect polymorphisms between rice individuals of the *japonica* and *indica* lines depending on whether the amplification products have a recognition site for restriction enzyme KpnI; and
(9) marker 9: PCR marker S12564 Tsp509I (SEQ ID NO:26) which, when rice genomic DNA is subjected to PCR with DNA primers having the sequences of SEQ ID NO:17 and SEQ ID NO:18, can detect polymorphisms between rice individuals of the *japonica* and *indica* lines depending on whether the amplification products have a recognition site for restriction enzyme Tsp509I.

Assuming that the locus of Rf-1 gene was highly likely to be located near the nine RFLP marker regions R1877, G291, R2303, S12564, C1361, S10019, G4003, S10602 and G2155 on chromosome 10 of rice (see the results of RFLP linkage analysis described in Fukuta et al., 1992, Jpn. J. Breed. 42 (supl. 1) 164-165 and the RFLP linkage map of rice described in Harushima et al., 1998, Genetics, 148, 479-494), the present inventors converted those RFLP markers to CAPS markers or dCAPS markers as described below in Example 1 (they are co-dominant PCR markers and described in Michaels and Amasino, 1988, The Plant Journal, 14(3), 381-385 and Neff et al., 1988, The Plant Journal, 14(3), 387-392). As a result of this conversion, PCR markers 1-9 were obtained.

These PCR markers can be classified into two groups, one consisting of R1877 EcoRI, G291 MspI (SEQ ID NO:22), R2303 BslI (SEQ ID NO:23) and S12564 Tsp509I (SEQ ID NO:26) and the other group consisting of C1361 MwoI (SEQ ID NO:20), S10019 BstUI (SEQ ID NO:24), G4003 HindIII (SEQ ID NO:19), S10602 KpnI (SEQ ID NO:25) and G2155 MwoI (SEQ ID NO :21). The PCR markers of the first group and those of the second group are on opposite sides of the locus of Rf-1 gene on chromosome 10 of rice . Therefore, in a preferred embodiment of the invention, at least one PCR marker selected from group (a) consisting of R1877 EcoRI, G291 MspI, R2303 BslI and S12564 Tsp509I, as well as at least one PCR marker selected from group (b) consisting of C1361 MwoI, S10019 BstUI, G4003 HindIII, S10602 KpnI and G2155 MwoI are used to detect Rf-1 linked bands, thereby detecting the presence of Rf-1 gene. In this case, at least S12564 Tsp509I is preferably selected from group (a) and at least C1361 MwoI from group (b) for use as PCR markers that are the closest in position to the Rf-1 gene. If the genome of the rice under test is found to contain two Rf-1 linked bands, one detected with a PCR marker of group (a) and the other with a PCR marker of group (b), it can be estimated with an extremely high probability that the rice contains Rf-1 gene.

In another embodiment of the invention, Rf-1 linked bands are detected using at least two PCR markers of group (a) and at least two PCR markers of group (b). For example, if one selects a rice individual that has Rf-1 linked bands detected by means of markers of groups (a) and (b) closer in position to Rf-1 gene on the gene map shown in Fig. 1 but which has no Rf-1 linked band detected by means of markers farther from Rf-1 gene than the first-mentioned markers, the selected rice individual has Rf-1 gene but contains the least possible amount of unwanted gene regions. Again, it is preferred that at least one PCR marker of group (a) is preferably S12564 Tsp509I and at least one PCR marker of group (b) is C1361 MwoI. These two PCR marker loci S12564 Tsp509I and C1361 MwoI are separated by a genetic distance of only 0.3 cM. By utilizing this characteristic, the chromosomal region that is introduced from the Rf-1 gene donor parent can be narrowed down to a size of about 1 cM. This helps minimize the possibility of introducing into the restorer line a deleterious gene that may be present in the neighborhood of Rf-1 gene in the donor parent.

### Primers

In another aspect of the invention, the following primer pairs are provided for use in amplifying PCR markers 1∼9. These primer pairs were also obtained according to the procedures described in Example 1.
(1) Primer pair 1: The primer pair for amplifying PCR marker R1877 EcoRI has the nucleotide sequences of SEQ ID NO:1 and SEQ ID NO:2.
(2) Primer pair 2: The primer pair for amplifying G4003 HindIII has the nucleotide sequences of SEQ ID NO:3 and SEQ ID NO:4.
(3) Primer pair 3: The primer pair for amplifying C1361 MwoI has the nucleotide sequences of SEQ ID NO:5 and SEQ ID NO:6.
(4) Primer pair 4: The primer pair for amplifying G2155 MwoI has the nucleotide sequences of SEQ ID NO:7 and SEQ ID NO:8.
(5) Primer pair 5: The primer pair for amplifying G291 MspI has the nucleotide sequences of SEQ ID NO:9 and SEQ ID NO:10.
(6) Primer pair 6: The primer pair for amplifying R2303 BslI has the nucleotide sequences of SEQ ID NO:111 and SEQ ID NO:12.
(7) Primer pair 7: The primer pair for amplifying S10019 BstUI has the nucleotide sequences of SEQ ID NO:13 and SEQ ID NO:14.
(8) Primer pair 8: The primer pair for amplifying S10602 KpnI has the nucleotide sequences of SEQ ID NO:15 and SEQ ID NO:16.
(9) Primer pair 9: The primer pair for amplifying S12564 Tsp509I has the nucleotide sequences of SEQ ID NO:17 and SEQ ID NO:18.

Using these primer pairs, one can amplify and detect the corresponding PCR markers from rice genome. It should be noted that slightly longer nucleotide sequences that contain the nucleotide sequences specified above or those which are slightly shorter can also be used as primers within the scope of the invention.

### Detection of the Rf-1 gene

In order to detect Rf-1 gene in the genome of a rice under test by the method of the invention, either one of PCR markers 1-9 is amplified from the genome of the rice by PCR using the appropriate primers of the invention and then detected by the polymerase chain reaction-restriction fragment length polymorphism method (PCR-RFLP).

PCR-RFLP is a method that is applicable to the case where polymorphisms exist among variety lines at recognition sites of restriction enzymes in the sequences of PCR amplified DNA fragments and by which specific polymorphisms can conveniently be identified on the basis of cleavage patterns with those restriction enzymes (D.E. Harry et al., Theor. Appl. Genet. (1998), 97:327-336).

The genomic DNA of a rice under test that is to be used as a template in PCR can be easily extracted by the method of Edwards et al. (Nucleic Acids Res. 8(6):1349, 1991). It is more preferred to use DNA as purified by standard techniques. The CTAB method (Murray, M.G. et al., Nucleic Acids Res. 8(19):4321-5, 1980) is a particularly preferred method of extraction. The DNA to be used as a template for PCR has preferably a final concentration of 0.5 ng/µL.

The procedures of PCR are well known in the art. By way of example, 50 ng of template genomic DNA, 200 µM of each dNTP and 5 U of ExTaq™ (commercially available from TAKARA) may be used to perform PCR comprising one cycle of 94 °C x 2 min, 30 cycles each consisting of 94 °C x 1 min, 58 °C x 1 min and 72 °C x 2 min, and finally one cycle at 72 °C x 2 min. Alternatively, one may perform PCR comprising one cycle of 94 °C x 2 min, 30 cycles each consisting of 94 °C x 1 min, 58 °C x 1 min and 72 °C x 1 min, and finally one cycle at 72 °C x 2 min. In another embodiment, one may perform PCR consisting of one cycle at 94 °C x 2 min, 30 cycles each comprising treatments at 94 °C x 30 sec, 58 °C x 30 sec and 72 °C x 30 sec, and finally one cycle at 72 °C x 2 min.

In order to check for restriction fragment length polymorphisms in the amplification product, the product is cleaved with the restriction enzyme corresponding to the restriction site present in the PCR marker selected from markers 1-9. For cleavage, the amplification products were incubated for periods ranging from several to about 24 hours at the reaction temperatures recommended for the specific restriction enzymes to be used. The amplified PCR sample cleaved with the restriction enzyme can be analyzed by performing electrophoresis on ca. 0.7% - 2% agarose gel or a ca. 3% MetaPhor™ agarose gel. The gels may then be visualized with ethidium bromide under ultraviolet illumination.

The presence or absence of the bands is confirmed on the gel in association with the primer pairs used as indicated below.

**Table 1**

| | Approximate size (bp) of detected band |
|---|---|
| Detection of marker 1 (R1877 EcoRI) with primer pair 1 | |
| When the genome of test rice has Rf-1 gene homozygously | 1500 and 1700 |
| When the genome of test rice has Rf-1 gene heterozygously | 1500, 1700 and |
| | 3200 |
| When the genome of test rice has no Rf-1 gene | 3200 |

| Detection of marker 2 (G4003 HindIII) with primer pair 2 | |
|---|---|
| When the genome of test rice has Rf-1 gene homozygously | 362 |
| When the genome of test rice has Rf-1 gene heterozygously | 95, 267 and 362 |
| When the genome of test rice has no Rf-1 gene | 95 and 267 |

| Detection of marker 3 (C1361 MwoI) with primer pair 3 | |
|---|---|
| When the genome of test rice has Rf-1 gene homozygously | 50 and 107 |
| When the genome of test rice has Rf-1 gene heterozygously | 25, 50, 79 and 107 |
| When the genome of test rice has no Rf-1 gene | 25. 50 and 79 |

| Detection of marker 4 (G2155 MwoI) with primer pair 4 | |
|---|---|
| When the genome of test rice has Rf-1 gene homozygously | 25, 27 and 78 |
| When the genome of test rice has Rf-1 gene heterozygously | 25, 27, 78 and 105 |
| When the genome of test rice has no Rf-1 gene | 25 and 105 |

| Detection of marker 5 (G291 MspI) | |
|---|---|
| with primer pair 5 | |
| When the genome of test rice has Rf-1 gene homozygously | 25, 49 and 55 |
| When the genome of test rice has Rf-1 gene heterozygously | 25, 49, 55 and 104 |
| When the genome of test rice has no Rf-1 gene | 25 and 104 |

| Detection of marker 6 (R2303 Bs1I) with primer pair 6 | |
|---|---|
| When the genome of test rice has Rf-1 gene homozygously | 238, 655 and 679 |
| When the genome of test rice has Rf-1 gene heterozygously | 238, 655, 679 and 1334 |
| When the genome of test rice has no Rf-1 gene | 238 and 1334 |

| Detection of marker 7 (S10019 BstUI) with primer pair 7 | |
|---|---|
| When the genome of test rice has Rf-1 gene homozygously | 130, 218 and 244 |
| When the genome of test rice has Rf-1 gene heterozygously | 130, 218, 244 and 462 |
| When the genome of test rice has no Rf-1 gene | : 130 and 462 |

| Detection of marker 8 (S10602 KpnI) with primer pair 8 | |
|---|---|
| When the genome of test rice has | |
| Rf-1 gene homozygously | 724 |
| When the genome of test rice has Rf-1 gene heterozygously | 117, 607 and 724 |
| When the genome of test rice has no Rf-1 gene | 117 and 607 |

| Detection of marker 9 (S12564 Tsp509I) with primer pair 9 | |
|---|---|
| When the genome of test rice has Rf-1 gene homozygously | 41 and 117 |
| When the genome of test rice has Rf-1 gene heterozygously | 26, 41, 91 and 117 |
| When the genome of test rice has no Rf-1 gene | 26, 41 and 91 |

### Advantages of the Invention

(1) Since the PCR-RFLP method is employed in the invention, the DNA marker to be detected is amplified by PCR, so it can be detected in a short time and conveniently without performing Southern blot analysis. Another advantage is that PCR-RFLP can be performed using only a few nanograms of DNA, which is extremely smaller than the amount required in RFLP when it involves Southern blot analysis. The DNA to be used need not be purified to a very high level and even a crude DNA extract may be used as such. In the present invention, PCR-RFLP may be used in combination with sequencing techniques in order to ensure the detection of the markers of interest more positively.

(2) The PCR markers of the invention are all co-dominant markers. Co-dominant markers can discriminate a heterozygote from a dominant homozygote. When it is required to determine the genotypic segregation ratio of F1 individuals produced by crossing heterogenotes, a dominant marker will simply enable us to discriminate individuals having the dominant gene and those having the recessive gene at a ratio of 3:1. On the other hand, if a co-dominant marker is used, dominant homozygotes, heterozygotes and a recessive homozygotes will be detected at a ratio of 1:2:1. Therefore, by using two of the nine co-dominant markers 1-9 that are located on opposite sides of the locus of Rf-1 gene, the genotype of Rf-1 to be introduced into hybrid rice can be correctly determined as to whether it is homozygous or heterozygous (i.e. whether it is Rf-1/Rf-1, Rf-1/rf-1 or rf-1/rf-1).

In the case of performing an analysis using, for example, PCR markers S12564 Tsp509I and C1361 MwoI, if the locus of Rf-1 gene in the plant is heterozygous, one allele carries the Rf-1 gene linked to both the region of marker S12564 that cannot be cleaved with restriction enzyme Tsp509I and the region of marker C1361 that cannot be cleaved with restriction enzyme MwoI, whereas rf-1 gene on the other allele is linked to both marker S12564 Tsp509I which can be cleaved with restriction enzyme Tsp509I and marker C1361 MwoI which can be cleaved with restriction enzyme MwoI. Therefore, by performing PCR on each of the two markers S12564 and C1361, two amplification products corresponding to polymorphisms at the site of restriction enzyme Tsp509I will be obtained with regard to S12564 whereas two amplification products corresponding to polymorphisms at the site of restriction enzyme MwoI will be obtained with regard to C1361. Hence, if each pair of amplification products is digested with the corresponding restriction enzyme, restriction fragment patterns will be presented that differ from those obtained in the case where the locus of Rf-1 gene is homozygous.

(3) PCR marker loci S12564 Tsp509I and C1361 MwoI are located on opposite sides of the locus of Rf-1 gene on chromosome 10, and the genetic distance between the loci of Rf-1 gene and S12564 Tsp509I is as short as 0.1 cM (equivalent to about 30 kbps) while the distance between Rf-1 and C1361 MwoI is about 0.2 cM short (equivalent to about 60 kbps). Considering the chiasma interference which teaches that when a crossing-over occurs at one site during meiosis, another crossing-over is less likely to occur in the surrounding region, there is little likelihood that double crossover events occur between the two markers that are spaced apart by a short distance of 0.3 cM. Therefore, by investigating the genotypes of the two marker loci, the locus of Rf-1 gene located between those marker loci can be correctly genotyped.

In this specification, the unit of genetic distance is the centiMorgan (cM); 1 cM represents a genetic distance that provides a cross-over frequency of 1%, or a genetic distance such that 1% of all gametes undergo recombination between two genetic loci. The probability of recombination varies greatly with species, so the base pair number that corresponds to 1 cM also varies with species. In the case of the rice, a main target of the invention, 1 cM is known to be equivalent to about 300 kbps of DNA on average although the exact number depends on the position on chromosome.

(4) As already mentioned, the two PCR marker loci S12564 Tsp509I and C1361 MwoI that are disclosed herein and which are closely linked to the locus of Rf-1 gene are spaced apart by a distance of only 0.3 cM. This character can be used to ensure that a chromosomal region introduced from the Rf-1 gene donor parent can be narrowed to the size of 1 cM or smaller. As a result, it is possible to exclude or reduce the potential risk of introducing a deleterious gene to the restorer line concomitantly with Rf-1 gene, even in the case that the donor parent has such a deleterious gene nearby to the Rf-1 gene. Therefore, by using the PCR marker loci S12564 Tsp509I and C1361 MwoI of the invention, the possibility of introducing a deleterious gene can be reduced to less than 1/30 compared with the case of using conventional marker loci G291 and G127 which are spaced by a distance of more than 30 cM on the map.

(5) In another embodiment, the PCR markers of the invention can be used to discriminate between rice varieties, *japonica* and *indica.* As already mentioned, the PCR markers of the invention can distinguish between the two rice varieties by making use of polymorphisms in the nucleotide sequences of restriction enzyme sites. Distinguishing *japonica* from *indica* rice is important, because if rice seeds of one variety contaminated with another variety are cultivated, rice of the desired variety cannot be harvested. According to the method of the invention, PCR markers can be amplified using only a few nanograms of DNA, so precise identification can be made for each grain of rice seed.

The present invention is described below in greater detail with reference to examples. It should be noted that the following examples are provided for illustrative purposes only and are in no way to be taken as limiting.

### Example 1: Conversion of RFLP markers around Rf-1 gene to PCR markers

In Example 1, nine RFLP markers (i.e., R1877, G291, R2303, S12564, C1361, S10019, G4003, S10602 and G2155) around the locus of Rf-1 gene were converted to PCR markers.

### (1) Materials and methods

The following nine RFLP markers, R1877, G291, R2303, S12564, C1361, S10019, G4003, S10602 and G2155, were purchased from the National Institute of Agrobiological Sciences, the Ministry of Agriculture, Forestry and Fisheries of Japan. After determining the nucleotide sequences of the inserts in the vectors, experiments were conducted according to the following procedures. While five rice varieties were used in the experiments, *Asominori, Koshihikari* and *Nipponbare* were *japonica,* and *IR24* and *Kasalath* were *indica.*

### (2) Preparation of Asominori genomic library

Total DNA was extracted from green leaves of *Asominori* by the CTAB method. After partial digestion with MboI, the DNA was fractionated according to size by NaCl density gradient centrifugation (6-20% linear gradient, 20 °C, 37000 rpm, 4 hr, total volume = 12 mL). A portion of each fraction (ca. 0.5 mL) was subjected to electrophoresis and fractions containing 15-20 kb DNA were collected and purified. A library was prepared using Lambda DASH II (Stratagene) as a vector in accordance with the attached protocol. Giga Pack III Gold (Stratagene) was used in packaging. After packaging, 500 µL of SM Buffer and 20 µL of chloroform were added. After centrifugation, 20 µL of chloroform was added to the supernatant to make a library solution.

XL-1 Blue MRA (P2) was infected with 5 µL of a 50-fold dilution of the library solution, whereupon 83 plaques were formed. This corresponded to 4.15 x 10⁵ pfu per library, and hence, it was calculated that the plaques covered 8.3 x 10⁹ bp assuming that the average length of the inserted fragments was 20 kb. The library was therefore considered to have an adequate size for the rice genome (4 x 10⁸ bp).

### (3) Isolation of genomic clones containing R1877-, C1361- and G4003-marker regions

As for C1361 and G4003, plasmids containing the RFLP marker probe were isolated and subjected to restriction enzyme treatment and electrophoresis to separate the RFLP marker probe portion; the desired DNA was recovered on a DNA recovery filter (Takara SUPREC-01). As for R1877, primers were designed that were specific to both ends of the marker probe and PCR was performed with the total DNA of *Asominori* used as a template; the amplification products were electrophoresed and recovered by the already-described method. The recovered DNA was labelled with a Rediprime DNA Labelling System (Amersham Pharmacia) to prepare a probe for screening the library. PCR was performed in the usual manner (this also applies to the following description).

Screening of the library was performed in the usual manner after blotting the plaques onto Hybond-N+ (Amersham Pharmacia). After primary screening, areas of positive plaques were individually punched out, suspended in SM buffer and subjected to the second round of screening. After the second screening, the positive plaques were punched out and subjected to the third round of screening to isolate a single plaque.

The isolated plaque of interest was suspended in SM buffer and primary multiplication of the phage was performed by the plate lysate method. The resulting phage-enriched solution was subjected to secondary multiplication by shake culture and the phage DNA was purified with Lambda starter kit (QIAGEN).

For each marker, primary screening was conducted on eight plates. A 10-µL aliquot of the library solution was employed per plate. After the primary, second and third rounds of screening, four genomic clones in association with R1877 were isolated and three were isolated in association with each of C1361 and G4003.

### (4) Conversion of R1877 to PCR marker

The isolated genomic clones were analyzed to identify the causative site of RFLP, or the EcoRI site that exists in IR24 but not in *Asominori*, thereby converting R1877 to a PCR marker.

The four isolated clones were subjected to the following analyses. First, T3 and T7 primers were used to determine the nucleotide sequences at both ends of the insert in each clone. Then, primers extending outwardly from both ends of the marker probe were designed. They were combined with T3 and T7 primers to give a combination of four primers in total, and employed in PCR with each clone used as the template.

In a separate step, each clone was digested with NotI and EcoRI and electrophoresed to estimate the insert size and the length of each EcoRI fragment.

These analyses revealed the relative positions of the individual clones. In RFLP analysis, marker probe R1877 was reported to detect an EcoRI fragment of 20 kb in *Nipponbare* and one of 6.4 kb in *Kasalath*
(ftp://ftp.staff.or.jp/pub/geneticmap98/parentsouthern/chr10/R 1877.JPG). This fact, taken together with the results of analysis described above, gave a putative position for the EcoRI site that existed in IR24 but not in *Asominori*. Hence, a primer combination (SEQ ID NO:1 x SEQ ID NO:2) that was designed to amplify the nearby region was employed to perform genomic PCR over 30 cycles, each cycle consisting of 94 °C x 1 min, 58 °C x 1 min and 72 °C x 2 min. The PCR product was treated with EcoRI and subjected to electrophoresis on 0.7% agarose gel. As a result, the expected polymorphisms were observed between *Asominori* and IR24. By treatment with EcoRI, the PCR product (-3200 bp) was cleaved to yield 1500 bp and 1700 bp fragments in IR24 but not in *Asominori.* Mapping of the marker was made with an RIL (recombinant inbred line) of *Asominori*-IR24 with the results that the PCR marker was located in the same region as that of RFLP marker locus R1877, thereby confirming the conversion of RFLP marker R1877 to a PCR marker, which was named R1877 EcoRI in the present invention.

### (5) Conversion of G4003 to PCR marker

The isolated genomic clones were analyzed to identify the causative site of RFLP, or the HindIII site that existed in *Asominori* but not in IR24, thereby converting G4003 to a PCR marker.

By performing analyses similar to those employed for R1877, the relative positions of the three isolated clones were revealed. In RFLP analysis, marker probe G4003 was reported to detect a HindIII fragment of 3 kb in *Nipponbare* and one of 10 kb in *Kasalath*
(ftp://ftp.staff.or.jp/pub/geneticmap98/parentsouthern/chr10/R 1877.JPG). This report, taken together with the analyses described above, led to a temporary conclusion that the HindIII site that existed in *Asominori* but not in IR24 would be at either one of two candidate sites. Hence, a primer combination that was designed to amplify the area in the neighborhood of each HindIII site was employed to perform genomic PCR over 35 cycles, each cycle consisting of 94 °C x 30 sec, 58 °C x 30 sec and 72 °C x 30 sec. The PCR product was treated with HindIII and subjected to electrophoresis on 2% agarose gel. As a result, the HindIII site within the marker probe was found to have polymorphisms. By treatment with HindIII, the PCR product (362 bp) was cleaved to yield a 95 bp fragment and a 267 bp fragment in *Asominori* but not in IR24. The primers used to amplify the polymorphic site were SEQ ID NO:3 and SEQ ID NO:4. Mapping of the site demonstrated the conversion of RFLP marker G4003 to a PCR marker, which was named *G4003* HindIII (SEQ ID NO:19) in the present invention.

### (6) Conversion of C1361 to PCR marker

Primers were designed on the basis of the nucleotide sequence information of the isolated genomic clones. PCR was performed with the total DNAs of *Asominori*, *Koshihikari, Kasalath* or IR24 being used as a template and the PCR product was recovered by known methods after electrophoresis. Using the recovered DNA as a template, the inventors analyzed the nucleotide sequence of each of the rice varieties with ABI Model 310 in search of mutations that would cause polymorphisms.

By performing analyses similar to those employed for R1877, approximate relative positions of the three isolated clones could be established. As it turned out, however, regions around the C1361 marker would be difficult to amplify by PCR or determine their nucleotide sequences, and hence, it would not be easy to identify the causative site of RFLP. Hence, the inventors took notice of the region capable of yielding a comparatively long PCR product (2.7 kb) and made an attempt to create a dCAPS marker. Upon comparing the nucleotide sequences of the genomic PCR products of said region using *Asominori, Koshihikari, Kasalath* and IR24, the inventors found six sites of polymorphism between *japonica* and *indica*. One of these six sites was used to create a dCAPS marker. To this end, with SEQ ID NO:5 and SEQ ID NO:6 used as primers, PCR was performed over 35 cycles, each cycle consisting of 94 °C x 30 sec, 58 °C x 30 sec and 72 °C x 30 sec. The PCR product was treated with MwoI and analyzed by electrophoresis on 3% MetaPhor™ agarose gel. In *Asominori*, cleavage occurred at two sites to give three observable bands of about 25 bp, 50 bp and 79 bp, but in IR24 cleavage occurred at one site to give two observable bands of about 50 bp and 107 bp. Mapping demonstrated the conversion of RFLP marker C1361 to a PCR marker, which was named C1361 MwoI (SEQ ID NO:20) in the present invention.

### (7) Conversion of G2155 to PCR marker

Primers specific to both ends of the marker probe were designed and PCR was performed with the total DNA of *Asominori*, *Koshihikari,* IR24 or IL216 (a line produced by introducing Rf-1 gene into *Koshihikari* by back crossing; its genotype was Rf-1/Rf-1) being used as a template.
Purification of the PCR product and searching for a mutation that would be useful for providing restriction fragment polymorphisms were performed by the methods already described above.

As a result of comparing the nucleotide sequences of corresponding regions of the varieties under test, mutations were found at three sites between the variety/line (IR24 or IL216) having Rf-1 gene and the variety (*Asominori* or *Koshihikari*) not having Rf-1 gene. One of the three sites was utilized to create a dCAPS marker. To this end, SEQ ID NO:7 and SEQ ID NO:8 were used as primers to perform PCR over 35 cycles, each cycle consisting of 94 °C x 30 sec, 58 °C x 30 sec and 72 °C x 30 sec. The PCR product was treated with MwoI and analyzed by electrophoresis on 3% MetaPhor™ agarose gel. In *Asominori,* cleavage occurred at one site to give two observable bands of about 25 bp and 105 bp, but in IR24, cleavage occurred at two sites to give three observable bands of about 25 bp, 27 bp and 78 bp. Mapping demonstrated the conversion of RFLP marker G2155 to a PCR marker, which was named G2155 MwoI (SEQ ID NO:21) in the present invention.

### (8) Conversion of G291 to PCR marker

Primers specific to internal sequences of the marker probe were designed and used in various combinations to perform PCR to find a primer combination that could yield an amplification product of the expected size. Using the selected primer combination, the inventors performed PCR with the total DNA of *Asominori, Koshihikari*, IR24 or IL216 used as a template. Purification of the PCR product and searching for a mutation that could be utilized in providing restriction fragment polymorphisms were performed by the methods already described above.

Using the primers designed to be specific for the marker probe sequence, the inventors performed genomic PCR of each variety under test and compared the nucleotide sequences of the products. As a result, mutations were found at four sites between the variety/line having Rf-1 gene (IR24 or IL216) and the variety *(Asominori* or *Koshihikari)* not having Rf-1 gene. One of the four sites was used to create a dCAPS marker. To this end, SEQ ID NO:9 and SEQ ID NO:10 were used as primers to perform PCR over 35 cycles, each cycle consisting of 94 °C x 30 sec, 58 °C x 30 sec and 72 °C x 30 sec. The PCR product was treated with MspI and analyzed by electrophoresis on 3% MetaPhor™ agarose gel. In the varieties/lines having Rf-1 gene, cleavage occurred at two sites to give three observable bands of about 25 bp, 49 bp and 55 bp, but in the varieties not having Rf-1 gene, cleavage occurred at one site to give two observable bands of about 25 bp and 104 bp. Mapping demonstrated the conversion of RFLP marker G291 to a PCR marker, which was named G291 MspI (SEQ ID NO:22) in the present invention.

### (9) Conversion of R2303 to PCR marker

Primers specific to internal sequences of the marker probe were designed and PCR was performed with the total DNA of *Asominori,* IR24 or *Kasalath* used as a template. Purification of the PCR product and searching for a mutation that could be used for providing restriction fragment polymorphisms were performed by the methods already described above.

As a result of comparing the nucleotide sequences of corresponding regions of the varieties under test, a mutation was found between *japonica* rice and *indica* rice. Since the mutation occurred at the BslI recognition site, the site was directly used to create a CAPS marker. To this end, SEQ ID NO:11 and SEQ ID NO:12 were used as primers and PCR was performed over 30 cycles, each cycle consisting of 94 °C x 1 min, 58 °C x 1 min and 72 °C x 2 min. The PCR product was treated with Bs1I and analyzed by electrophoresis on 2% agarose gel. In *japonica* rice, cleavage occurred at one site to give two observable bands of about 238 bp and 1334 bp, but in *indica* rice, cleavage occurred at two sites to give three observable bands of about 238 bp, 655 bp and 679 bp. Mapping demonstrated the conversion of RFLP marker R2303 to a PCR marker, which was named R2303 BslI (SEQ ID NO:23) in the present invention.

### (10) Converting S10019 to PCR marker

S10019 was converted to a PCR marker in accordance with the method of converting R2303 to a PCR marker.

As a result of comparing the nucleotide sequences of corresponding regions of the varieties under test, a mutation was found between *japonica* rice and *indica* rice. Since the mutation occurred at the BstUI recognition site, the site was directly used to create a CAPS marker. To this end, SEQ ID NO:13 and SEQ ID NO:14 were used as primers and PCR was performed over 30 cycles, each cycle consisting of 94 °C x 1 min, 58 °C x 1 min and 72 °C x 1 min. The PCR product was treated with BstUI and analyzed by electrophoresis on 2% agarose gel. In *japonica* rice, cleavage occurred at one site to give two observable bands of about 130 bp and 462 bp, but in *indica* rice, cleavage occurred at two sites to give three observable bands of about 130 bp, 218 bp and 244 bp. Mapping demonstrated the conversion of RFLP marker S10019 to a PCR marker, which was named S10019 BstUI (SEQ ID NO:24) in the present invention.

### (11) Conversion of S10602 to PCR marker

S10602 was converted to a PCR marker in accordance with the method of converting R2303 to a PCR marker.

As a result of comparing the nucleotide sequences of corresponding regions of the varieties under test, a mutation was found between japonica rice and *indica* rice. The mutation was used to create a CAPS marker. To this end, SEQ ID NO:15 and SEQ ID NO:16 were used as primers and PCR was performed over 33 cycles, each cycle consisting of 94 °C x 1 min, 58 °C x 1 min and 72 °C x 1 min. The PCR product was treated with KpnI and analyzed by electrophoresis on 2% agarose gel. In *japonica* rice, cleavage occurred at one site to give two observable bands of about 117 bp and 607 bp, but in *indica* rice, no cleavage occurred, giving only an observable band of 724 bp. Mapping demonstrated the conversion of RFLP marker S10602 to a PCR marker, which was named S10602 KpnI (SEQ ID NO:25) in the present invention.

### (12) Conversion of S12564 to PCR marker

S12564 was converted to a PCR marker in accordance with the method of converting R2303 to a PCR marker.

As a result of comparing the nucleotide sequences of corresponding regions of the varieties under test, a mutation was found between *japonica* rice and *indica* rice. The mutation was used to create a dCAPS marker. To this end, SEQ ID NO:17 and SEQ ID NO:18 were used as primers and PCR was performed over 35 cycles, each cycle consisting of 94 °C x 30 sec, 58 °C x 30 sec and 72 °C x 30 sec. The PCR product was treated with Tsp509I and analyzed by electrophoresis on 3% MetaPhor™ agarose gel. In *japonica* rice, cleavage occurred at two sites to give three observable bands of 26 bp, 41 bp and 91 bp, but in *indica* rice, cleavage occurred at one site to give two observable bands of 41bp and 117 bp. Mapping demonstrated the conversion of RFLP marker S12564 to a PCR marker, which was named S12564 Tsp509I (SEQ ID NO:26) in the present invention.

### Example 2: Mapping of Rf-1 Gene Locus

DNA was extracted from 1042 seedlings of the F1 population produced by pollinating MS *Koshihikari* with MS-FR *Koshihikari*, and the DNA extract was used in the analysis. MS *Koshihikari* (generation: BC10F1) was created by replacing the cytoplasm of *Koshihikari* with BT type male sterility cytoplasm. MS-FR *Koshihikari* was a line created by introducing Rf-1 gene from IR8 into MS *Koshihikari* (the locus of Rf-1 gene being heterozygous).

First, each individual was investigated for the genotype at two marker loci R1877 EcoRI and G2155 MwoI that would presumably be located on opposite sides of the locus of Rf-1 gene. *Japonica* type homozygotes with respect to either locus R1877 EcRI or G2155 MwoI were regarded as recombinants between these two marker loci. Then, each of such recombinants was investigated for the genotypes of G291 MspI, R2303 Bs1I, S12564 Tsp 509I, C1361 MwoI, S10019 BstUI, G4003 HindIII and S10602 KpnI loci, and the positions of recombination were identified.

The genotype investigation with respect to R1877 EcoRI and G2155 MwoI loci revealed that 46 individuals were recombinants around the locus of Rf-1 gene. Genotypes of the marker loci around the locus of Rf-1 gene were investigated and the results are shown in Table 2. Based on the results of the crossing study, and in consideration of the previous report showing that in individuals having BT type cytoplasmic male sterility, only the pollen having Rf-1 gene is capable of fertilization (C. Shinjyo, JAPAN. J. GENETICS, Vol. 44, No. 3: 149-156 (1996)), the locus of Rf-1 gene could be located on the precise linkage map as shown in Fig. 1.

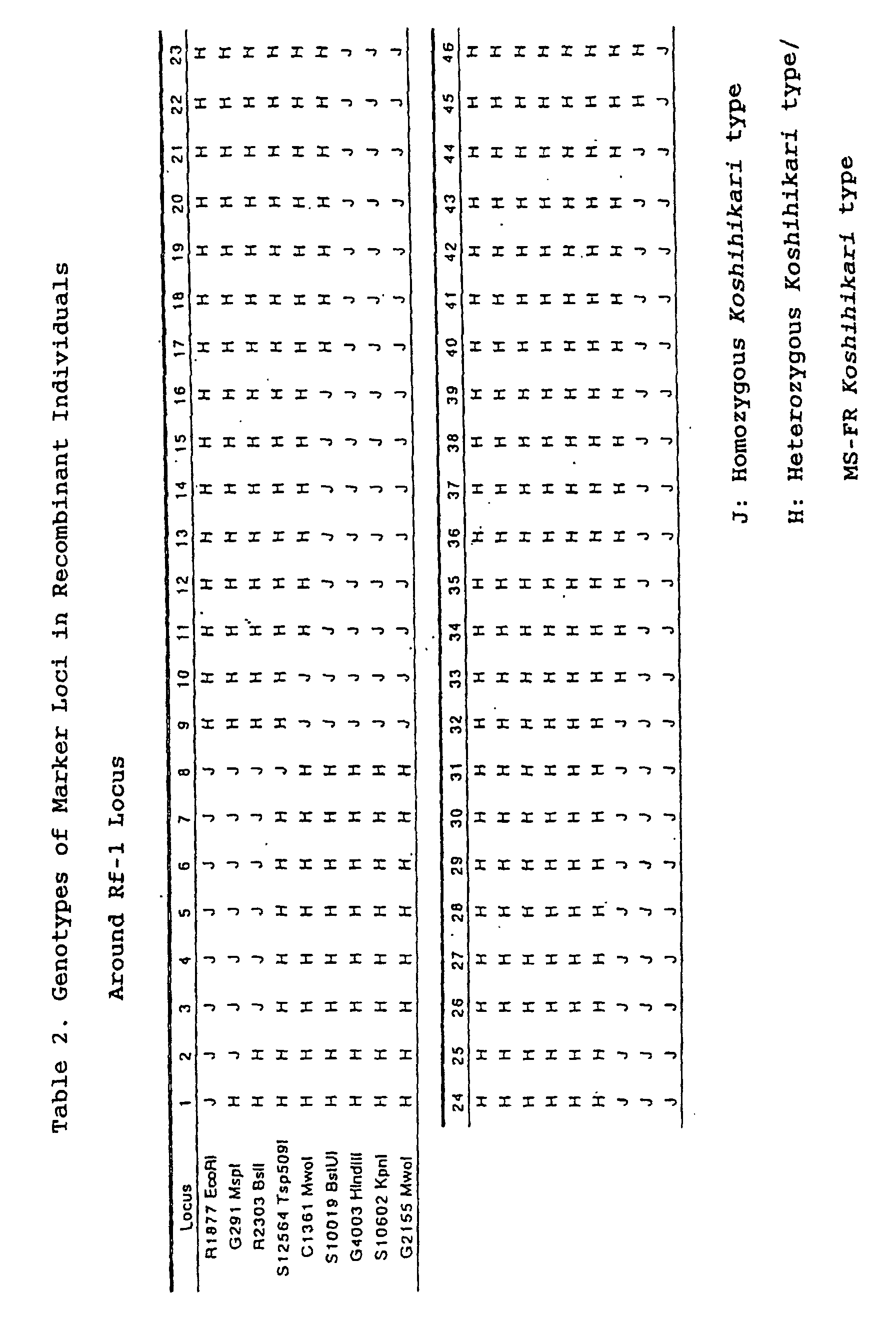

In order to genotype the marker loci that would be closely linked to the locus of Rf-1 gene, the present inventors developed nine novel co-dominant PCR markers including two novel co-dominant PCR markers S12564 Tsp509I and C1361 MwoI located on opposite sides of the locus of Rf-1 gene. The nine novel co-dominant PCR markers provide a convenient and accurate method for genotyping the locus of Rf-1 gene. By using the PCR markers of the invention, investigation for the presence or absence of Rf-1 gene which restores the BT type cytoplasmic male sterility currently used in the breeding of *japonica-japonica* hybrid rice and selection of Rf-1 gene homozygotes could be performed in a convenient and accurate manner. Therefore, the PCR markers of the invention can be used in efficient breeding of hybrid rice. As a further advantage, the method of the invention enables direct genotype estimation from DNA sequences and, hence, can genotype the locus of Rf-1 gene without being influenced by environmental factors.

Sequence Listing

<110> JAPAN TOBACCO INC.

<120> A method for genotyping the locus which is involved in

restoration of the rice BT type cytoplasmic male sterility.

<130> 991698

<160> 26

<210> 1

<211> 24

<212> DNA

<213> artificial sequence

<223> Oligonucleotide primer for amplification of R1877 EcoRI marker

sequence.

<400> 1

cattcctgct tccatggaaa cgtc 24

<210> 2

<211> 33

<212> DNA

<213> artificial sequence

<223> Oligonucleotide primer for amplification of R1877 EcoRI marker

sequence.

<400> 2

ctctttctgt atacttgagc tttgacatct gac 33

<210> 3

<211> 20

<212> DNA

<213>  artificial sequence
<223>  Oligonucleotide primer for amplification of G4003 HindIII marker
sequence.
<400>  3
gatcgacgag tacctgaacg                                                    20

<210>  4
<211>  24
<212>  DNA
<213>  artificial sequence
<223>  Oligonucleotide primer for amplification of G4003 HindIII marker
sequence.
<400>  4
aatagttgga ttgtcctcaa aggg                                               24

<210>  5
<211>  27
<212>  DNA
<213>  artificial sequence
<223>  Oligonucleotide primer for amplification of C1361 MwoI marker
sequence.
<400>  5
aaagcaaccg acttcagtgg catcacc                                            27

<210>  6
<211>  24
<212>  DNA
<213>  artificial sequence
<223>  Oligonucleotide primer for amplification of C1361 MwoI marker

sequence.
<400> 6
ctggacttca tttccctgca gagc 24

<210> 7
<211> 27
<212> DNA
<213> artificial sequence
<223> Oligonucleotide primer for amplification of G2155 MwoI marker
sequence.
<400> 7
gaccaccaat taactgatta agctggc 27

<210> 8
<211> 27
<212> DNA
<213> artificial sequence
<223> Oligonucleotide primer for amplification of G2155 MwoI marker
sequence.
<400> 8
tttctggctc caataatcag ctgtagc 27

<210> 9
<211> 27
<212> DNA
<213> artificial sequence
<223> Oligonucleotide primer for amplification of G291 MspI marker
sequence.
<400> 9

ctgctgcagc aagctgcacc gaaccgg 27

<210> 10
<211> 24
<212> DNA
<213> artificial sequence
<223> Oligonucleotide primer for amplification of G291 MspI marker
sequence.
<400> 10
   acattttttc ttccgaaact tccg 24

<210> 11
<211> 24
<212> DNA
<213> artificial sequence
<223> Oligonucleotide primer for amplification of R2303 BslI marker
sequence.
<400> 11
atggaaagat acactagaat gagc 24

<210> 12
<211> 24
<212> DNA
<213> artificial sequence
<223> Oligonucleotide primer for amplification of R2303 BslI marker
sequence.
<400> 12
atcttatata gtggcaggaa agcc 24

<210> 13

<211> 24

<212> DNA

<213> artificial sequence

<223> Oligonucleotide primer for amplification of S10019 BstUI marker

sequence.

<400> 13

aacaatctta tcctgcacag actg 24

<210> 14

<211> 24

<212> DNA

<213> artificial sequence

<223> Oligonucleotide primer for amplification of S10019 BstUI marker

sequence.

<400> 14

gtcacataga agcagatggg ttcc 24

<210> 15

<211> 24

<212> DNA

<213> artificial sequence

<223> Oligonucleotide primer for amplification of S10602 KpnI marker

sequence.

<400> 15

agctgttgag agttctatgc cacc 24

<210> 16

<211> 24

<212> DNA
<213> artificial sequence
<223> Oligonucleotide primer for amplification of S10602 KpnI marker
sequence.
<400> 16
tagccatgca acaagatgtc atac 24

<210> 17
<211> 26
<212> DNA
<213> artificial sequence
<223> Oligonucleotide primer for amplification of S12564 Tsp509I marker
sequence.
<400> 17
ctagttagac cgaataactg aggttc 26

<210> 18
<211> 27
<212> DNA
<213> artificial sequence
<223> Oligonucleotide primer for amplification of S12564 Tsp509I marker
sequence.
<400> 18
tttgtgggtt tgtggcattg agaaaat 27

<210> 19
<211> 2240
<212> DNA
<213> Oryza sativa L.

<223> PCR marker G4003 HindIII

<400> 19

gcggccgctc cgggaagtcg agcgagtaga cgcccctgac gccgtacgcg tcggcgagcc 60

gcagcggcgt ctctggcggt gtgaaggaca gcccgttcag cgtcgcgcgg cgccgcccgt 120

tgatcgtcac cggcgccgtg ctccgcagca ggtacgcctg cgtcacgttg atcgacgagt 180

acctgaacga tccctgtggg ttcggcctcg ccgctccggc actcaggttc cacctgccca 240

atgcaaaaaa ccaaaaccca aaagcttaat gcgaataata catcattcca cgtatttaaa 300

aaaataattt ataggtaaaa tttttataat gtattttagc gacgtaaatg tcaatgctga 360

gaaataaacg ataatacttt aaatgaagtt ctaaaattta aattttggca tcggttgatg 420

ttggataaag aaaacgatgg aggctagtaa tttttcttct tttttaagta tctagattgt 480

catatattga atttttcagt ttttcatccc tttgaggaca atccaactat tattttcctt 540

ttcttatgta aaaggttgaa caacatattc aaacataaaa aaataaaatt aaatgaaata 600

aatttacaat tcataaaatt tacagaattt atgttaagaa aatattcaaa cttagataat 660

aataaagcaa caaaatcgta ctaaaaagaa gtataattgt acattgtata ctactactcc 720

tacaatttta gacttagaat ttttaatttc ctgaaatcta gtaatgccat ttttttcttt 780

ctagttgaac cagacagtaa gtttaactcg aaacttataa gctaatgagc gaagtcgggc 840

aattcactcg tacctgacgg agcgagcttg gttcatggag aaggacttgt cgaactggtc 900

ctggggaggg tcggggagcg ggccggaggc ccgcccccgg gagttggagt agcggaggac 960

ggcgacgccg gcgacgcggc gccacacggt gtcgttcacc atgcgcgcgc tggcgacgac 1020

gtagtagtcg gagctcgcgt tctggtcggt ggtgacgagg aaggagtagg actggccgac 1080

gtggacgtcc aggttggtgt agttctgctg cgtcgtgtag gagccctccg tctccaccag 1140

caccatgttg tgcccctgga tcctgaagtt gaggctcgtc gacgtcccca cgttgtgcac 1200

tcggatcctg tacgtcttgc ctgtgtcccc acaccgacgt cgccgacaca cgcgcaaaag 1260

ataatagact cattgtaagt aggtagtaac cttctccgtt tcatattata aatcgtttga 1320

ttatattttt gttagttaaa cttctttaag ttttttttct ataaacttaa ttaaatctaa 1380

agaattttaa taaaaaaaat caaacgactt ataatataaa atggatggag tagttgcatc 1440

aatttgtgga tgaagcaaac aagattatat ccttttcatg agggtgaaag tattcagtga 1500

acaattcgtc agtttcaagt ttcatgaaat cggacagggt ctctgaaagt ctgtattttt 1560

ggtactgttg gattgactac tctggcttct gttgtcacat cttttgtatc ctagtttcgg 1620

taaaaaaaat tttggcattt ttactcctat cgttgatctg tttaactgaa accattgcat 1680
gatatactac tagcagacaa aactggtgaa aattcacgag aatgaacttt ttgtcagtta 1740
agcattagcg gacagcttca gtaagcagag caggctgcct taaggcttaa agcactatct 1800
tccacaacac tttgtcctac aatcaaattc caaatttact atcacaaaaa gcgaaggaac 1860
taactaaacc ttactcctac tagtactact gctatgacta tgaaacaaga ttccaatcca 1920
aagaaaacac agtgctcgat cagcatgata aaagcaacga aacctgctca tccagctgcc 1980
aaaatgccac cccactgact ctacgtacgt actacgtatt gacgctgtaa aaaactagcc 2040
gtagtacaga gaagaggacc caaagtttcg tcaaaaattt tattttaccc ggatccacat 2100
tgatggtctc gtactcgatg ccggccggga caaggctgtc gttgtacctg tacgggccct 2160
tgccgttaat cagcacgccg tccggcatcc cgaggtcctt gccactgtcc agcatcttcc 2220
tcagatcctg caacgaattc 2240

<210> 20
<211> 2601
<212> DNA
<213> Oryza sativa L.
<223> PCR marker C1361 MwoI
<400> 20
tcttgctgag atccaagttg cggtaacttt gcccttttct ttttttcttc tcttctgaat 60
tttttcatgg tttttgggag agattttcgt aacttgatta cagttctagg aaaaggccac 120
cttgttcaaa cagggctttc ttgaaaggga tcaatttgct aggagtacat gattctaaaa 180
gcgatttcga aataaaacac agttctcgat ctcatacctg aaaacaaaag gcccatactg 240
tgtaaactgt gattatgctt ctgttaaatg ggatatttgt acaaaattga cgccaaccac 300
ctataaacag attgtgagct tttatcttag taaaataaaa tgtgacattc tactcagtgt 360
tcagtgatcc gatgtcgtct cttctgcgta caacttctaa cagccgtttt cggtagtaca 420
aactagcgaa acaccaaaaa cgcagcattt gagttctgga atacgctgaa attgttagaa 480
tcaaccacga aaccaaaatc attgttcaga aacgttgcaa cgagataaaa cacaagaact 540
tgttttaaca aagcatacgg acagtacata tacggttaca acacccagtc tttatacagt 600
tctgctggag ttccatctac tggctgtcat tgtatctcag gacagacagg ttaacatagg 660

tacaacacaa ttacaggcta aaccgaagcg aactacactg tcagcatctc taacagtatc 720
gtcaagcaag cttatttaca gctgctctag taaatttaca acgtccctgg cagaatccct 780
ctcgtttctg gcagcgacga ggcacggtcc atggccttag caggacatct cacccgtcag 840
ctgcatagaa agcaaccgac ttcagtggaa tcacctcctg ctcctgcaaa aaagttggtt 900
cgatcaatca cgcgtttaat ccaaaacaaa atgggtatta attatgctag cctatgaagc 960
tacctcagag ttctctattt gctctgcagg gaaatgaagt ccagtggaac agttctcaag 1020
cacctcaggg ctcttcatcc atgctttgtg tgcttcaatg gctttcagct tatagcgaaa 1080
catctgcgat acggatctaa aattaaggat gtcgacaatt acttaacaca acaaataatt 1140
gaagcaggtc cagttaaaga aaagtagcag cgaagaatag cactctgaag tctgaacctc 1200
agataaagaa atggttggtt tttccagttc atctccctca acatggattc cagtaccctg 1260
gcattctggg caaaggatgg atgttatttt cttaggtgca ttttttgcct ttcttcctcg 1320
attgcttttt cccttgcttg caattttgtc tgctagcatc tcatattggc ataaaatagt 1380
ccagtgcaca aggcaagaag tgtgaaacaa atgaaatgcc tgcaaaatta gccgtacaaa 1440
gtcattggag gttgcagcag aatactacaa atttttaaag aagaaactat acactgtcta 1500
tgttttgctt gaaatgaatt caaccacttt gcattatacg gtttggaatc cctggtttgt 1560
gagaactgta attccattac aacagtgaag aagttaccat aactaatgaa tggaaattag 1620
tcaaatgcct aattttttag gtttgcttta atttatttat ctgtgagaaa tgctaagcat 1680
gtcatgcgtt gctatcttca agaaatacta agaaactgca aaggcaaaga atgtttgaaa 1740
taacttaccc cgcttgagtt tctactgctg caggctagat ttcctgtctt gcagttgagc 1800
aaggtagcta catccttttc aagaagcatt ggtcgcccac aaatatcaca agctttctca 1860
gcagcaaggc gcttctgctt acgcaactcc ctcctcatag atttggtgga taagaggcca 1920
acttgaagat tgtgtgaagt acctgtcggg gaacctgtta tgatagcttg gctattgtca 1980
tgggcggagc tgctttgctc attcgactcc tctgaagatg cttcttgatc tgaaaatgac 2040
ttctttcttc tctttccacg gtgtccagca tcatcaatca cgaagaaaga tccagcagag 2100
ataggaaggt cctgatcatc agaagaccac ttcctgccca actcaattgt ataagagaag 2160
ttgacaatgg caaagtcaga ttgctcatag gtgtcacact catccaagcc atgggagcca 2220
tcctgtccta cccaagcaca ccagatcttg ctaatctttt tacttccttt gctagcttcc 2280
cataacctgt atgcaatatt tccatatccc aaaagatgca caggcaaatc cgaaacaaca 2340
tcctttagca atacactagg aataacgaga ggaccgtcag ttccactttg gtttgacagc 2400

acatgatctt cagatacaga agcagttcta ccattaccat gcgcatttgc accacggcgt 2460
gtgccttttg cgccattgcg agagctagaa tcatctctca acctcgaagt cacttcagtg 2520
tcgttcgctg gaaccagagc cagctctctg gtgttctgcg agctcgagtc cagcaagagc 2580
gggtccttct cgcgcgagtt g 2601

<210> 21
<211> 1333
<212> DNA
<213> Oryza sativa L.
<223> PCR marker G2155 MwoI
<400> 21
ccctctgctt gatccagtgt acatccatgg gttaggacag attagttact cagttaatta 60
agtgtgagac tggaaaaaaa tatctgacgg cagttttata agttgagtga ttgaactagt 120
gaaagttcag ttaactgtca acggctgtag atttgggatg gcagactgtt ctgagtcaaa 180
atgaagcttt tactgtgcgt ggttaccagg tgcagtaaaa taatttcaga tctaatcgca 240
gtaaaaaaat gtagtactat atgttaagac gagattggtc ggtcaaaatc tatctggccc 300
tttacatctc ccaaatgtta cctcagttgc aggtggtaaa aaaaaatcac tcgtttcacg 360
tgatgtcggc agatcatgga ccatgtctca aatgctgaaa ctctgaacaa tcaacaaaaa 420
aatccaacca gatgagctgt gcaactgata attgatcatc acactatttg caactcatct 480
ttcatgtaga tggaacttca atcccgaaga aataatgaca gcaaaatgct gcgatcctga 540
agaaaggatg gcggcaaaat ggcagcgata aaaaaaaaat ggttggttac tgaagaatta 600
tttgtgcagc agttgagaca gtagcaagat aagagctagc taagctagct aggtagagtt 660
ggatggaaga gtagtagtat gagatagagc atggagcgcg acaactcaag tggatgctaa 720
agtaaaaggc attctcttct cttgtttgga atcagaaaag aaaagaaaag acttgagctg 780
cttggctgga atgtttggtt ggatcatgcg cgctctcctt agcttagctc gccaagaaat 840
cctcgcttca tctctctcaa taattcaaag ccacgagctc tctgctcata tccagtgcga 900
cgattcccgt taatgcaaat gcattatatc cagttcgaaa tgttacaatt cttgcgtttg 960
cagcaagcca gcaagtggtg tgaattgttt aatccctcgt gcatttcaac gaaattctct 1020
cacaaattcg cattgacttc tttcttagca caattagtaa gcagtgacaa ataaagaatt 1080

tttgaacagg atgtctttcc aaggaaggtg agatttttta tgtggatagc aaggatcgcc 1140
tttccttagc atgaagagaa tgtgatcaac tttacacctt gcttacgatt atggccttaa 1200
tttttgatac cctaaacagg agcacatcac atgcatgtcg acctgagacc accaattaac 1260
tgattaagtt ggcatttcag atgcatccgt cagttacatg atcaggtgat cgatggatca 1320
actgtaggtt tca 1333

<210> 22
<211> 863
<212> DNA
<213> Oryza sativa L.
<223> PCR marker G291 MspI
<400> 22
cgaacaggat caaaagtaga cgacgagggc atttagaagg agaggaattg tatttgttcc 60
cggtatttaa tttttaaatt tgtggtcgga agtttcggaa gaaaaaatgt gctcatgagt 120
gattattggc tctgaacacc aacctctctt ttcgttgatt ccttctgagg tgttgggtgt 180
tgggacacga tgctgccgcc gacacgacac cgggttccac aatacactaa tctactcgcg 240
acaccttcat tgaactgcat ataattattt agaaagtcca ttaacacatc ttataaaacc 300
ttgttgaatc atataatcat tctataaagt ctatttgaac atcttatgaa aaaataagat 360
ctgacctagt cgttacactc tcttacattt tccattagcc taactaattc cgtgcaggaa 420
acgcccaaaa ataatagtac caatagtcca ctaatcccgt gccagaggcc gccaatgatt 480
agtgattaac ccaaaaaaca taatcatcat cacacgccgc taatgaccag ctctcgctta 540
gctcatccca caggcggccc ccacacgcca ctcctgccat gtgggcccac ctttcacacc 600
ccccaccaac cagaaaaaaa actcccccaa aaaaaaaact tttaatgctt atctcgcggc 660
agtataaaag gcgaccccac cacccacaca caatcacagt cagcgaccca acccaacccg 720
agccgaggag tcgagtcgtg tgaaaattac gaaattgccc ttcgactcca ccaccaccac 780
ccaccggcga ggcgaggaga ggagaaaaat tgggaggaaa aaaaaaggga aaaagaaaaa 840
gggtggagga gatttttgcg aag 863

<210> 23

<211> 1510

<212> DNA

<213> Oryza sativa L.

<223> PCR marker R2303 BslI

<400> 23

tgccatgaag acctatggaa agaatatctt cttctcactc tgtgaatggt gagtttactc 60

tctgtaacat ttagggctag gtcgaaggaa catgaagcat tgctgattca ctccactgtg 120

tttttttttt ctgtataggg ggaaagaaaa tcctgctaca tgggcaggcc gcatgggtaa 180

cagctggaga acaactggcg acatcgccga caactggggc aggttctact catcctctct 240

ttaaccctgt ttacatagtt cttgagtttt tcagtactga tcgtaattgc cctgttattt 300

cagtatgaca tctcgtgcag acgaaaatga ccaatgggct gcctatgctg gacctggtgg 360

atggaatggt aagaacttga gatgtatctg ttcctaggtt gcttaaccat ttgagagctt 420

caaaatgatc aacatatgtt tctgctgtgc aatatcagat cctgacatgc ttgaagtggg 480

aaatggtggg atgtctgaag ctgagtaccg gtcacacttc agtatctggg cactagcaaa 540

ggtaccatag catgttctat gtactaataa ttttgctgca atgttgaact tctttgcatt 600

tcctcactgc aagttttgct tgaattgttc aggctcctct tttgatcgga tgcgatgtgc 660

gctcaatgag ccagcagacg aagaacatac tcagcaactc ggaggtgatc gctgtcaacc 720

aaggcaagcc ttctcagttt cacatgctta gatttagcca tacctcttgg atatttcacc 780

atactcataa tgtaactctc tgaacagata gtctaggtgt ccaaggaaag aaagtacaat 840

ctgacaacgg attggaggta tcccttcaat ggcttccaaa tttgcagttt ctcattgtcc 900

cataagcctt ggcatgatca tgactaactc tgaagctgac aatactttgt gtaaatttgt 960

cggtaggttt gggccgggcc actcagcaac aacaggaagg ctgtggtgct ctggaacagg 1020

cagtcatacc aggcaaccat cactgcacat tggtcgaaca tcgggctcgc tggatcggtc 1080

gcggtcactg ctcgtgatct atgggcggta aagcctttgc tttcttcaga gctcaaagta 1140

gaacatcttc tcttcagaat tcagagttca taacaaattt ctgtcaattg tgcagcactc 1200

ttcgttcgcg gctcagggac agatatcagc atcggtggcg cctcatgact gcaagatgta 1260

tgtcttgaca ccaaactagt cagcaaagaa aagcagcaca ggttagtacg tgtccggcga 1320

atacagctaa attgatcagg attcaggaag aaggtttgca atttgcaagg attggtagag 1380

ctggaaatgg gatgccattt ggttatgtat gtagaaataa gctgtaagcc tgtaagcgta 1440

tatgtaatca gccgtcaaat gctggcgagt gtatttctga agtttgcaac gaaagttgca 1500
gcaataaaaa 1510

<210> 24
<211> 1016
<212> DNA
<213> Oryza sativa L.
<223> PCR marker BstUI
<400> 24
tggggattct tttctttaag caatttaaca ttattgtcct aacaatatac acaatattgg 60
tttttctttc agtatcaaat aattctttta cttttgaaaa cacatttgca atgtgttgga 120
aacacaatta tatcttgcac ttccttttgg aaatttaatc atttgaaaac tgattcgcgt 180
ttcatggctg taatcttctc ttgcgaacat cgctctttct ttgatggttc tctgttgaga 240
agaagagcaa ccaagtaaat tttcgaaatg tttttttgtt ctttctattc accattgcag 300
gttgtcaaag ccatcgagaa ggccataccg attccgagag cgcaacccat tgccttggat 360
ggcccagcaa gggaagagct gaaggccatg gaggcgcaga aggtcgagat cgaccgcacc 420
gcggcgctcc aggtgcgccg tgagctttgg ctggggctgg catacctcgt cgtccagact 480
gccggcttca tgaggctcac attctgggag ctctcatggg atgtcatgga acccatctgc 540
ttctatgtga cctccatgta cttcatggcc ggctacacct tcttcctccg gaccaagaag 600
gagccctcct tcgagggctt cttcgagagc cggttcgcgg cgaagcagaa gcggttgatg 660
cacgcccggg atttcgatct ccgccggtat gacgagctcc ggcgagcctg tggcctgccg 720
gtggttcgga ctccgacgag cccctgcaga ccgtcgtcgt cgtcgtcgtc gtcttcgacg 780
caggagagcc attgccattc ttactgccat tgccaatgat ctttgtgctg ttctgttctg 840
ttgtcagaat tttttcatgc ccagtttatg ggggttaagc tagcttctcc attgtaccgt 900
tctgatgtgc ggatgatgcg atgcaaagca tagtttgttg aagagatgac aaggcagatt 960
ttagcttgaa aacctggagg tgagaaaaaa aaatcctgat gtgtttgtgt gtgtga 1016

<210> 25
<211> 676

<212> DNA
<213> Oryza sativa L.
<223> PCR marker S10602 KpnI
<400> 25
accaccttca tatgaagaaa ttaacggtgt tttcatgagg aatccaacag tcgctgaatt 60
ggtggaaact gtggaattct tcttggctga ggtaaccaat catcacttca ccacaatgca 120
caagtttgta gcttactact acagtacttc taataagttt tgtctgttga gattttattg 180
ctgatttcta tgcatggtca tctttttgac aggccatcca gtcttatcgt gctgagagtg 240
aaactgagct caacctggca gctggtgact atatagttgt ccggaaggta cggccctatc 300
ttcccattgg acatgtttct aaccataaac atatctttgc tggacttttg tgggcaaagt 360
tggctacact aaacttgtgt tcattaacct gctcaatcag gtgtcaaaca atggatgggc 420
agaaggtgaa tgcagaggga aagctggctg gttcccttac gactacatcg agaaaaggga 480
ccgtgtgctt gcaagtaaag tcgcccaggt cttctaggcg ttcaatgagc catacataca 540
taaccctggt gttgtacact gtattatgat cgttcgtgat cttcaaagac cctctgatca 600
gagaaatcac aaatattctt ttgttctatt attgtcatta tcactacccc ttttgtcaaa 660
accagtgcag cctttt 676

<210> 26
<211> 1059
<212> DNA
<213> Oryza sativa L.
<223> PCR marker Tsp509I
<400> 26
gcgagatcat gaacttgatt ttctggttgc catattgggc ttgcttgtta accttgtaga 60
gaaggatagc cttaataggt aagtccctca catgcttcct tccatttgct caattcatat 120
cagtgttact gttctggcag ttccttgggg tcaggactca gaaacatcca attaatgttc 180
atgttctctt aacgactcag aaatacttta taacctctcc acagggtacg gctttcatct 240
gcccgtgttc ctgttgatct atctcagaat ccacagagtg aagagacaca gagagatgtc 300
atagcactcc tctgttctgt attcttagca agtcaaggtg ctagtgaagc ttctggaact 360

atatcaccgg taattcaaaa ttcttcaagt tccttttgta tgtagattat atctttgtaa 420
aactcggcat ttattacctg ctctttgttt caaaaagcag tattttattt tgctccttag 480
cataggtcag cagaacagtt gatcttattc agaaaacaat attttgcatg taacatactg 540
ttatctatga gatgaaaatt aatgcatgtg taataatgtc aatgataaat atttgctatc 600
tgaatccagt ctaccaactc tagttagacc gaattactga ggttctattt caaagaataa 660
tttagtgcac catttgttca actactatga agtaaaatgg tattcccttc tattgacatc 720
gggttagaag tgaaaggcca tcttaatgcg atgttctcaa tgccacaaac ccacaaattt 780
cattaacaca tacagattat tattaacata gctataaatt ggatttccag aagcttgagt 840
tgaatttatt ttgttacaat tgaaagcact gggaacatta gcattttttt ttagttcttg 900
gttattgcaa tttataatgt tatacagaac tgtgtacctc acaatgcatt cattatgaca 960
ttctatgaac catttgattg actgttgctt gtaaacaaca ggatgatgag gagtctttga 1020
tgcaaggagc acgggaagct gaaatgatga tcgtagagg 1059

## Claims

1. A method of determining whether a rice individual or seed under test has Rf-1 gene based on the fact that the locus of a fertility restoring gene (Rf-1 gene) is located between RFLP marker loci S12564 and C1361 on chromosome 10 of rice.

2. The method according to claim 1, which detects at least one PCR marker selected from the group consisting of:
(1) PCR marker R1877 EcoRI which, when rice genomic DNA is subjected to PCR with DNA primers having the sequences of SEQ ID NO:1 and SEQ ID NO:2, can detect polymorphisms between rice individuals of the *japonica* and *indica* lines depending on whether the amplification products have a recognition site for restriction enzyme EcoRI;
(2) PCR marker G4003 HindIII which, when rice genomic DNA is subjected to PCR with DNA primers having the sequences of SEQ ID NO:3 and SEQ ID NO:4, can detect polymorphisms between rice individuals of the *japonica* and *indica* lines depending on whether the amplification products have a recognition site for restriction enzyme HindIII;
(3) PCR marker C1361 MwoI which, when rice genomic DNA is subjected to PCR with DNA primers having the sequences of SEQ ID NO:5 and SEQ ID NO:6, can detect polymorphisms between rice individuals of the *japonica* and *indica* lines depending on whether the amplification products have a recognition site for restriction enzyme MwoI;
(4) PCR marker G2155 MwoI which, when rice genomic DNA is subjected to PCR with DNA primers having the sequences of SEQ ID NO:7 and SEQ ID NO:8, can detect polymorphisms between rice individuals of the *japonica* and *indica* lines depending on whether the amplification products have a recognition site for restriction enzyme MwoI;
(5) PCR marker G291 MspI which, when rice genomic DNA is subjected to PCR with DNA primers having the sequences of SEQ ID NO:9 and SEQ ID NO:10, can detect polymorphisms between rice individuals of the *japonica* and *indica* lines depending on whether the amplification products have a recognition site for restriction enzyme MspI;
(6) PCR marker R2303 BslI which, when rice genomic DNA is subjected to PCR with DNA primers having the sequences of SEQ ID NO:11 and SEQ ID NO:12, can detect polymorphisms between rice individuals of the *japonica* and *indica* lines depending on whether the amplification products have a recognition site for restriction enzyme Bs1I;
(7) PCR marker S10019 BstUI which, when rice genomic DNA is subjected to PCR with DNA primers having the sequences of SEQ ID NO:13 and SEQ ID NO:14, can detect polymorphisms between rice individuals of the *japonica* and *indica* lines depending on whether the amplification products have a recognition site for restriction enzyme BstUI;
(8) PCR marker S10602 KpnI which, when rice genomic DNA is subjected to PCR with DNA primers having the sequences of SEQ ID NO:15 and SEQ ID NO:16, can detect polymorphisms between rice individuals of the *japonica* and *indica* lines depending on whether the amplification products have a recognition site for restriction enzyme KpnI; and
(9) PCR marker S12564 Tsp509I which, when rice genomic DNA is subjected to PCR with DNA primers having the sequences of SEQ ID NO:17 and SEQ ID NO:18, can detect polymorphisms between rice individuals of the *japonica* and *indica* lines depending on whether the amplification products have a recognition site for restriction enzyme Tsp509I.

3. The method according to claim 2 which concludes that the rice individual or seed under test has Rf-1 gene when at least one PCR marker selected from group (a) consisting of R1877 EcoRI, G291 MspI, R2303 BslI and S12564 Tsp509I and at least one PCR marker selected from group (b) consisting of C1361 MwoI, S10019 BstUI, G4003 HindIII, S10602 KpnI and G2155 MwoI are both present in the amplification products.

4. The method according to claim 3, wherein the presence of PCR markers S12564 Tsp509I and C1361 MwoI is detected.

5. A method of detecting a chromosomal region that has been introduced from an Rf-1 gene donor parent, which comprises genotyping loci around Rf-1 gene in an individual under test using at least two PCR markers in the same group of at least one of the groups (a) and (b) in claim 3.

6. A primer to be used in the method according to claim 2 which has the sequence of either one of SEQ ID NO:1 to SEQ ID NO:18.
